(19) Europäisches Patentamt

European Patent Office

Office européen des brevets

(11) **EP 1 062 977 B1**

(12) **EUROPÄISCHE PATENTSCHRIFT**

(45) Veröffentlichungstag und Bekanntmachung des Hinweises auf die Patenterteilung:
**26.10.2005 Patentblatt 2005/43**

(51) Int Cl.⁷: **A61N 1/368**, A61N 1/37

(21) Anmeldenummer: **00110871.1**

(22) Anmeldetag: **23.05.2000**

(54) **Verfahren zur Detektion kardialer Intervallsignale in kardiologischen Geräten**

Method of detecting intervals in cardiac signals within cardiological devices

Procédé de détection d'intervals dans des signaux cardiaques dans des appareils cardiologiques

(84) Benannte Vertragsstaaten:
**AT BE CH CY DE DK ES FI FR GB GR IE IT LI LU MC NL PT SE**

(30) Priorität: **23.06.1999 DE 19928656**

(43) Veröffentlichungstag der Anmeldung:
**27.12.2000 Patentblatt 2000/52**

(73) Patentinhaber: BIOTRONIK Mess- und Therapiegeräte GmbH & Co Ingenieurbüro Berlin
**12359 Berlin (DE)**

(72) Erfinder:
• **Hahn, Andreas, Dr.**
**10245 Berlin (DE)**

• **Kucher, Andreas, Dr.**
**16303 Schwedt (DE)**

(74) Vertreter: **Hübner, Gerd et al**
**Rau, Schneck & Hübner**
**Patentanwälte**
**Königstrasse 2**
**90402 Nürnberg (DE)**

(56) Entgegenhaltungen:
**EP-A- 0 663 218**  **US-A- 4 796 620**
**US-A- 5 527 344**

**Beschreibung**

Die Erfindung betrifft ein Verfahren zur Detektion kardialer Intervallsignale in kardiologischen Geräten, insbesondere zur Detektion von tachykarden Herzrhythmusstörungen mit den im Oberbegriff des Patentanspruches 1 angegebenen Verfahrensmerkmalen.

Aus einschlägigen bekannten technischen Geräten, nämlich beispielsweise Herzschrittmachern, die zur Bekämpfung von tachykarden Herzrhythmusstörungen eingesetzt werden, ist es bekannt, durch spezielle Detektionsverfahren kardiale Ereignisse des überwachten Herzens zu erfassen, die daraus gewonnenen Meßergebnisse entsprechenden Auswertekriterien zu unterwerfen und daraus mit geeigneten Algorithmen entsprechende Therapieabgaben in Form von elektrischen Impulsen an das überwachte Herz zu bestimmen. Als kardiale Ereignisse sind beispielsweise die bei der atrialen und ventrikulären Kontraktion des Herzens auftretenden Reizsignale heranzuziehen, die für das Herzintervall - also den zeitlichen Abstand zwischen zwei Herzkontraktionen - repräsentativ sind.

Die atrialen und ventrikulären Herzraten bzw. -intervalle und ihr Vergleich können u. a. als Kriterium für das Vorliegen tachykarder Herzrhythmusstörungen herangezogen werden.

In diesem Zusammenhang ist es in auswertungstechnischer Hinsicht bei kardiologischen Geräten nach dem Stand der Technik bereits bekannt, die zeitlich aufgelöst erfaßten, kardialen Ereignisse, die für die Herzintervalle repräsentativ sind, heranzuziehen, um das jeweilige Herzintervall zwischen zwei solchen aufeinanderfolgenden kardialen Ereignissen zu bestimmen. Eine bestimmte Anzahl aufeinanderfolgender Herzintervall-Werte werden dann statistisch ausgewertet, um eine gewisse Unterdrückung von Fehlmessungen zu erreichen. So wird beispielsweise eine sogenannte "x-aus-y-Kontrolle" vorgenommen, bei der definierte Ereignisse nur dann als Basis für die Bestimmung geeigneter Therapieabgaben im kardiologischen Gerät herangezogen werden, wenn sie x-mal innerhalb von y-Erfassungen aufgetreten sind.

Gängig ist im Zusammenhang mit der Detektion tachykarder Herzrhythmusstörungen ferner eine gleitende Mittelwertbildung für die statistische Auswertung, bei der eine bestimmte Anzahl aufeinanderfolgender Herzintervall-Werte einer laufenden Mittelwertbildung unterzogen werden. Werden in der Praxis atriale und ventrikuläre Ereignisse zur Herzintervall-Bestimmung herangezogen, so werden die laufend ermittelten atrialen und ventrikulären Mittelwerte der Herzintervall-Werte miteinander verglichen, wobei signifikante Abweichungen dieser Mittelwerte voneinander und definierte Verhalten aufeinanderfolgender Mittelwerte auf bestimmte Herzrhythmusstörungen schließen lassen. Entsprechend vorgegebener Algorithmen in der Steuerung des kardiologischen Gerätes werden dann auf der Basis der vorstehend beschriebenen Detektion entsprechende Therapieabgaben an das überwachte Herz ermittelt. Diese Abfolgen, die im Vorstehenden naturgemäß sehr abstrakt darzustellen waren, werden in praxi durch entsprechend programmierte Algorithmen in den Mikroprozessorgestützten Steuerungen moderner kardiologischer Geräte umgesetzt.

In der Anwendung können nun bei der Erfassung von kardialen Ereignissen durchaus Fehlmessungen auftreten. Beispielsweise ist bei der Detektion von atrialen und ventrikulären Ereignissen ein sogenanntes "Undersensing" oder "Oversensing" zu verzeichnen. Unter "Undersensing" ist das fehlerhafte Nicht-Feststellen von tatsächlich stattgefundenen atrialen oder ventrikulären Ereignissen zu verstehen. "Oversensing" ist eine Fehlmessung, bei der ein Impuls erfaßt wird, obwohl kein entsprechendes atriales oder ventrikuläres Ereignis stattgefunden hat.

Bei der Herzintervall-Bestimmung wirkt sich nun ein vereinzeltes atriales oder ventrikuläres "Undersensing" dahingehend aus, daß sich der daraus ermittelte Herzintervall-Wert verdoppelt. Bei einer statistischen Auswertung in Form einer Mittelwertbildung beispielsweise über vier aufeinanderfolgende Herzintervall-Werte geht nun der falsch gemessene Wert viermal in Folge in eine gleitende Mittelwertbildung ein. Insoweit wird also viermal in Folge das eigentliche Herzintervall falsch bestimmt, was - insbesondere im Wiederholungsfalle - zu einer inadäquaten Therapieabgabe seitens des kardiologischen Gerätes mit entsprechenden Gefahren für den Patienten führen kann.

Aus dem Dokument EP-A-0 663 218 ist ein Verfahren zur Fehlerdetektion und -korrektur bekannt, wobei der Herzintervallwert mit dem gleitenden Mittelwert von vorher verglichen wird und im Ergebnis dieses Vergleichs korrigiert wird.

Der Erfindung liegt demzufolge die Aufgabe zugrunde, das Verfahren zur Detektion kardialer Intervallsignale dahingehend zu verbessern, daß einzelne Fehlmessungen erkennbar und insbesondere auch im Hinblick auf die statistische Auswertung aufeinanderfolgender Herzintervall-Werte korrigierbar sind.

Diese Aufgabe wird dadurch gelöst, daß einhergehend mit der statistischen Auswertung der bestimmten Anzahl von aufeinanderfolgenden Herzintervall-Werten ein Einzelvergleich dieser bestimmten Anzahl von Herzintervall-Werten untereinander derart erfolgt, daß einzelne, von vorher und danach auftretenden Herzintervall-Werten signifikant abweichende Herzintervall-Werte als Fehlmessungen erkannt und entsprechend korrigiert werden.

Das erfindungsgemäße Verfahren macht sich dabei die Erkenntnis zu nutze, daß bei der Detektion kardialer Intervallsignale Fehlmessungen in aller Regel als sogenannte "Ausreißer", also als ausgeprägte Einzelereignisse auftreten. Ein solches Einzelereignis ist nun durch einen laufenden Vergleich aufeinanderfolgender erfaßter Herzintervall-Werte insbesondere in retrospektiver Betrachtung erkennbar und kann aufgrund der Speichermöglichkeiten der Mikroprozessor-gestützten Steuerungen moderner kardiologischer Geräte und Implantate zu einer entsprechenden Korrektur der Bewertung der für eine Therapiebestimmung zugrundeliegenden Herzereignisse herangezogen werden. Insofern können also bevorzugtermaßen bei einer überlagerten statistischen Auswertung und Korrektur der detektierten Herzintervall-Werte die bereits statistisch ausgewerteten Herzintervalle bei Erkennung einer Fehlmessung nachträglich korrigiert werden.

Bei einer statistischen Auswertung auf der Basis einer gleitenden Mittelwertbildung werden vorzugsweise mindestens vier aufeinanderfolgende Herzintervall-Bestimmungen herangezogen. Vorzugsweise erfolgt dann der Einzelvergleich der Herzintervalle untereinander zur Erkennung von Fehlmessungen mit einer Anzahl von Herzintervallen, die mit der für die Mittelwertbildung herangezogenen Anzahl von Herzintervall-Bestimmungen übereinstimmt.

Weitere bevorzugte Ausführungsformen des erfindungsgemäßen Verfahrens betreffen spezielle Verfahrensschritte für die Behandlung von Fehlereignissen bei dem bereits angesprochenen "Undersensing" bzw. "Oversensing" bei der Erfassung atrialer oder ventrikulärer Ereignisse. Näheres hierzu ergibt sich aus der folgenden Beschreibung eines Ausführungsbeispiels des erfindungsgemäßen Verfahrens anhand der beigefügten Zeichnung. Es zeigen:

Fig. 1 ein Meßdiagramm auf der Basis der zeitlich aufgelösten Erfassung von atrialen und ventrikulären Ereignissen,

Fig. 2 ein Schaubild zur Darstellung der laufenden Mittelwertbildung bei der statistischen Auswertung aufeinanderfolgender atrialer und ventrikulärer Herzintervall-Werte ohne erfindungsgemäße Korrektur,

Fig. 3 eine tabellenartige Übersicht zur Darlegung des laufenden Vergleiches zwischen Herzintervall-Mittelwerten von atrialen und ventrikulären Ereignissen bei atrialem "Undersensing",

Fig. 4 und 5 Diagramme analog Fig. 2 und 3 bei ventrikulärem "Undersensing",

Fig. 6 eine zu Fig. 2 und 3 analoge, kombinierte Darstellung eines ventrikulären "Oversensings", und

Fig. 7 eine zu Fig. 2 und 3 analoge, kombinierte Darstellung eines atrialen "Oversensings".

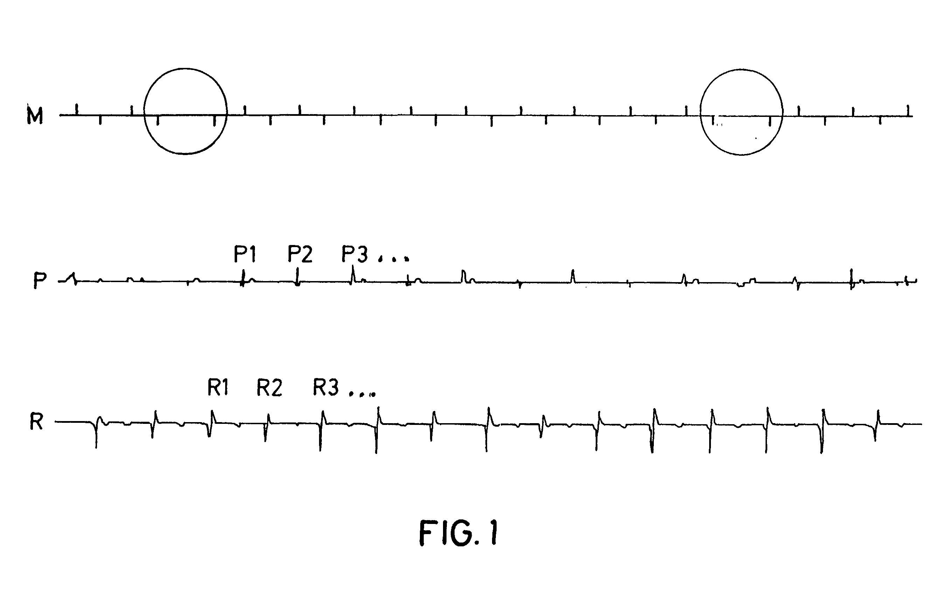

Anhand von Fig. 1 ist das erfindungsgemäße Detektionsverfahren am Beispiel eines vereinzelten atrialen "Undersensings" zu erläutern. Ausgangspunkt bildet das in Fig. 1 ausschnittsweise gezeigte EKG des Herzens, wie es von einem Anti-Tachykardie-Implantat zur Bekämpfung tachykarder Herzrhythmusstörungen aufgenommen wird. Als kardiale Ereignisse werden dabei einerseits die atrialen Reizsignale verwendet, die durch die sogenannte P-Welle des EKG repräsentiert werden (Zeile P in Fig. 1). Die Zeitabstände zwischen den einzelnen P-Wellen P1, P2, P3 ... bestimmen die atrialen Herzintervalle des überwachten Herzens.

Ferner werden als kardiales Ereignis die in Fig. 1 in Zeile R dargestellten R-Wellen R1, R2, R3 ... erfaßt, die die ventrikuläre Erregung des überwachten Herzens repräsentieren. Die Abstände zwischen den einzelnen R-Wellen R1, R2, R3 ... sind repräsentativ für die ventrikulären Herzintervalle des überwachten Herzens. Schließlich ist in Fig. 1 in Zeile M der sogenannte "Marker" des Anti-Tachykardie-Implantats dargestellt, der die Umsetzung der realen Signale der P- und R-Wellen im Implantat zu getriggerten Basissignalen für das zeitliche Auftreten der jeweiligen P- und R-Welle P1, P2, P3 ..., R1, R2, R3 ... wiedergibt.

In den eingekreisten Bereichen der Zeile M ist erkennbar, daß dort jeweils eine P-Welle aufgrund einer Fehlmessung nicht erfaßt und daher kein entsprechendes atriales Ereignis vom Implantat erkannt wird. Es hat also eine Fehlmessung in Form eines atrialen "Undersensings" stattgefunden.

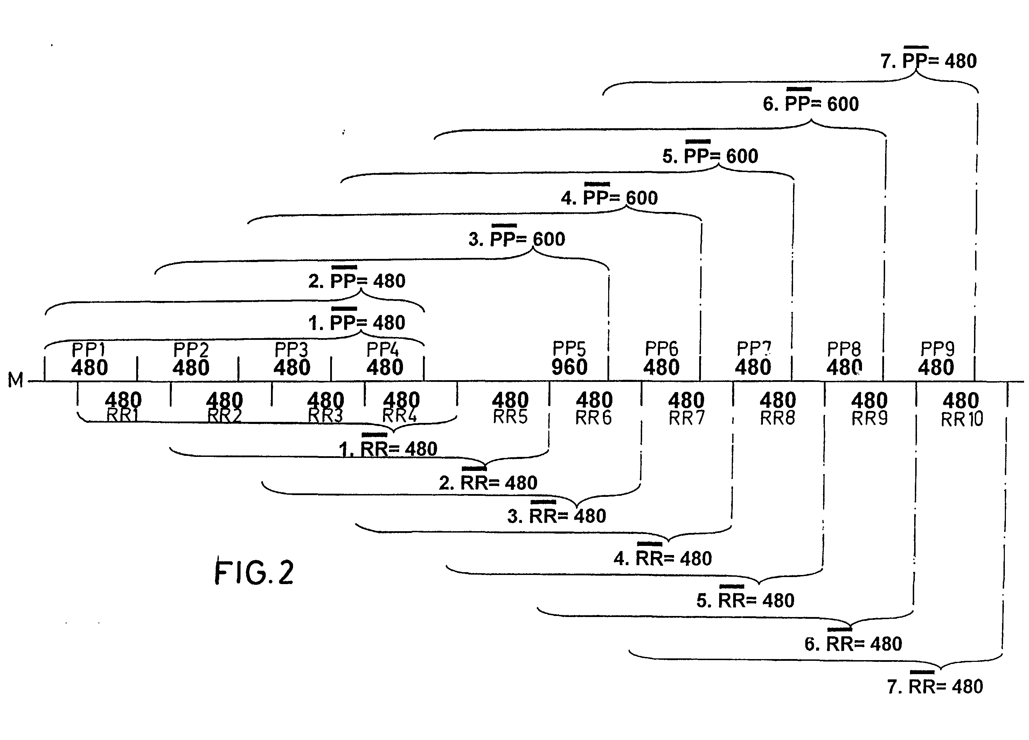

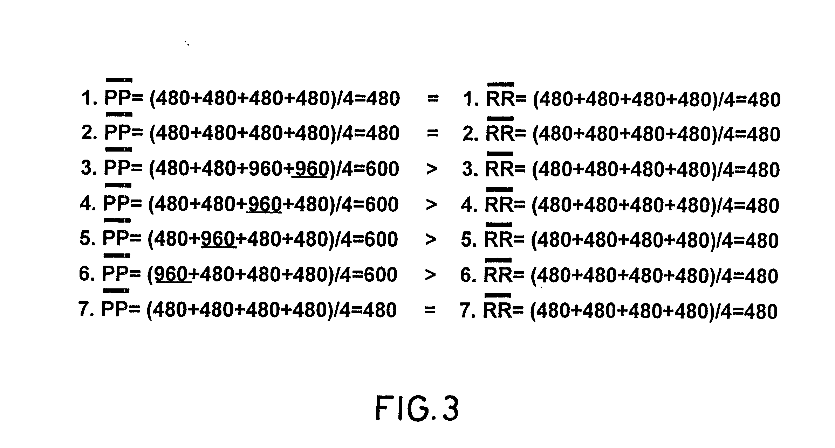

Die weitere statistische Auswertung der atrialen und ventrikulären Ereignisse und der Einfluß der vorstehend erwähnten Fehlmessung ist anhand der Fig. 2 und 3 zu erläutern. So werden jeweils aufeinanderfolgende Herzintervall-Werte zwischen zwei aufeinanderfolgenden atrialen bzw. ventrikulären Ereignissen aus dem Marker bestimmt. Wie aus der Zeile M in Fig. 2 hervorgeht, betragen die Herzintervall-Werte zwischen den atrialen P-Wellen jeweils 480 ms mit Ausnahme des Bereichs der Fehlmessung, wo sich eine Intervall-Länge von 960 ms (2 x 480 ms) ergibt.

Die zeitlich gegenüber den P-Wellen verschobenen R-Wellen, bei denen keine Fehlmessung stattgefunden hat, zeigen jeweils Herzintervalle von 480 ms.

Zur statistischen Auswertung findet nun eine laufende Mittelwertbildung über vier Herzintervall-Werte PP1, PP2, PP3, PP4, dann PP2, PP3, PP4, PP5, dann PP3, PP4, PP5, PP6 usw. statt. Analog wird für die ventrikulären Ereignisse eine gleitende Mittelwertbildung über jeweils vier ventrikuläre Herzintervall-Werte RR1, RR2, RR3, RR4, dann RR2, RR3, RR4, RR5 usw. vorgenommen. Es werden daraus entsprechende Mittelwerte 1. $\overline{PP}$, 2. $\overline{PP}$, 3. $\overline{PP}$ ... bzw. 1. $\overline{RR}$, 2. $\overline{RR}$, 3. $\overline{RR}$ usw. gebildet.

Wie sich nun aus Fig. 3 ergibt, ist durch die fehlerhafte Messung des atrialen Intervalls PP5 in Höhe von 960 ms bei vier aufeinanderfolgenden Mittelwerten, nämlich der 3. $\overline{PP}$ bis zum 6. $\overline{PP}$, ein falscher Mittelwert von 600 ms zu verzeichnen. Da der Mittelwert $\overline{PP}$ je-

weils mit dem zugehörigen Mittelwert $\overline{RR}$ des ventrikulären Ereignisses verglichen wird, wird bei den Vergleichen 3. $\overline{PP}$/3. $\overline{RR}$ bis 6. $\overline{PP}$/6. $\overline{RR}$ jeweils ein nicht den tatsächlichen Gegebenheiten entsprechendes Verhältnis zwischen den atrialen und ventrikulären gemittelten Herzintervall-Werten festgestellt. Diese falsche Feststellung kann zu der falschen Annahme führen, daß eine ventrikulär bedingte Tachykardie vorliegt, da das Herz - wie fälschlich angenommen wird - ventrikulär schneller schlägt als atrial.

Aufgrund des erfindungsgemäßen Verfahrens wird nun einhergehend mit der vorstehenden statistischen Auswertung auf der Basis einer gleitenden Mittelwertbildung aus vier aufeinanderfolgenden Intervallwerten auch ein Einzelvergleich jeweils vier aufeinanderfolgender Herzintervall-Werte vorgenommen. So werden beispielsweise während des Vergleiches 2. $\overline{PP}$/2. $\overline{RR}$ auch die vier atrialen Herzintervall-Werte PP1, PP2, PP3, PP4 miteinander verglichen. Da sie gleich groß sind, wird kein Fehler erkannt.

Beim anschließenden Vergleich 3. $\overline{PP}$/3. $\overline{RR}$ werden die atrialen Herzintervall-Werte PP2, PP3, PP4 und PP5 miteinander verglichen. Es wird festgestellt, daß sich PP5 signifikant von den vorherigen Werten PP2, PP3, PP4 unterscheidet, nämlich doppelt so groß ist. Im Steuerprogramm des Implantats wird ein entsprechender Vermerk gemacht.

Beim nächsten Vergleich 4. $\overline{PP}$/4. $\overline{RR}$ werden die atrialen Herzintervall-Werte PP3, PP4, PP5, PP6 miteinander verglichen und es wird festgestellt, daß der Herzintervall-Wert PP6 wieder auf den ursprünglichen Wert von PP3, PP4 zurückgegangen ist. Dies wird als Kriterium dafür erkannt, daß PP5 eine Fehlmessung - also ein "Ausreißer" - sein muß, was dazu führt, daß der Herzintervall-Wert PP5 korrigiert wird. Entsprechend werden reprospektive die Mittelwerte 3. $\overline{PP}$, 4. $\overline{PP}$, 5. $\overline{PP}$ und 6. $\overline{PP}$ zu 480 ms korrigiert, die entsprechenden Vergleiche 3. $\overline{PP}$/3. $\overline{RR}$, 4. $\overline{PP}$/4. $\overline{RR}$ usw. wiederholt und entsprechend neu bewertet. Dieser Vorgang, der in Fig. 2 und 3 nicht eigens aufgeführt ist, führt dazu, daß bei den Vergleichen $\overline{PP}$/$\overline{RR}$ jeweils atriale und ventrikuläre Mittelwerte bestimmt werden, die übereinstimmen, was zu der geänderten Bewertung führt, daß keine Tachykardie vorliegt.

Aus dem Vorstehenden wird deutlich, daß bei einem fehlerhaft nicht-festgestellten kardialen Ereignis, wie einem atrialen "Undersensing", genau ein Herzintervall-Wert PP5 signifikant von den vorher und nachher bestimmten Herzintervall-Werten PP4, PP6 abweichen muß, was zu einer Erkennung einer Fehlmessung und einer entsprechenden Korrektur dieses Wertes führt.

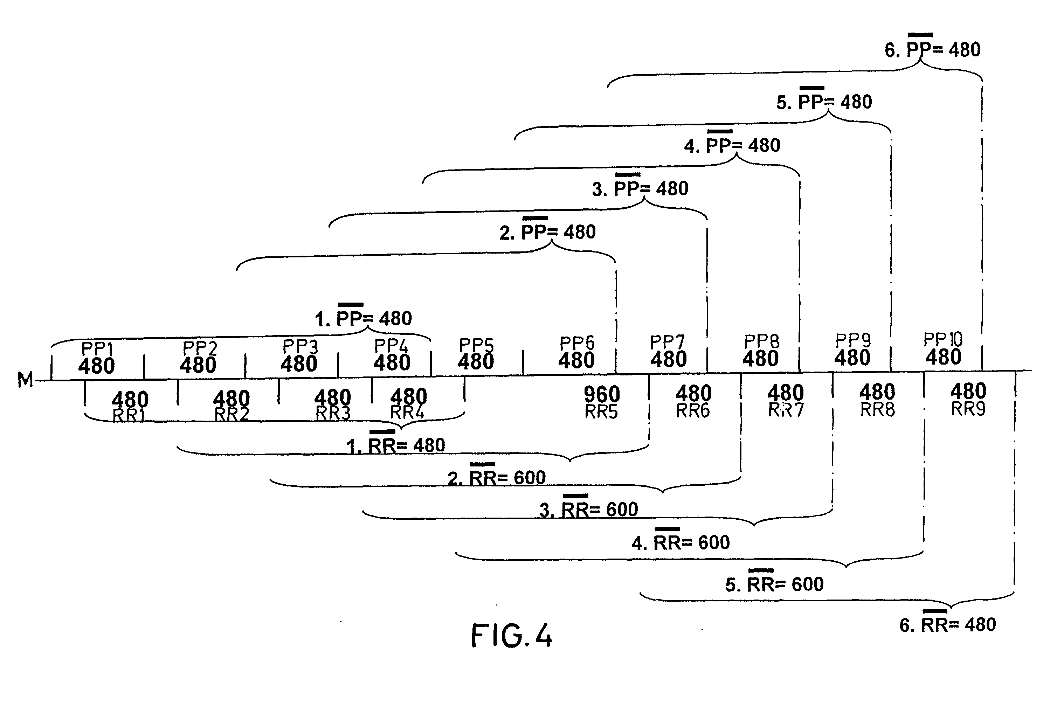

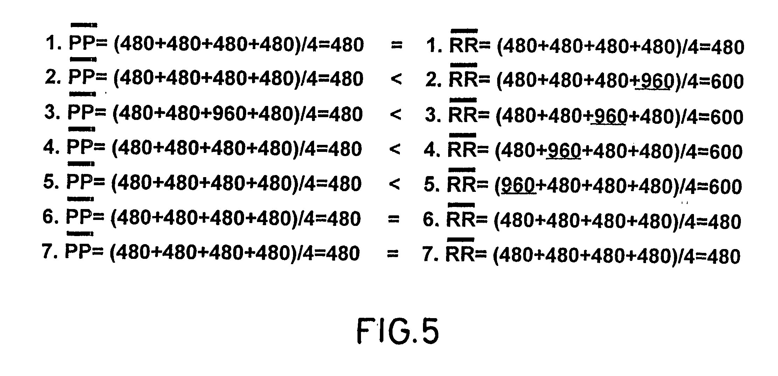

Aus den Fig. 4 und 5 ist ein vereinzeltes ventrikuläres "Undersensing" und seine Korrektur zu erläutern. Da die grundsätzlichen statistischen Auswertungsmethoden, nämlich eine gleitende Mittelwertbildung über vier aufeinanderfolgende Herzintervall-Werte PP1, PP2, PP3, PP4 bzw. RR1, RR2, RR3, RR4 usw. übernommen werden, braucht dies nicht nochmals eigens erläutert zu werden. Festzuhalten ist lediglich, daß sich durch eine Fehlmessung bei einer R-Welle der Herzintervall-Wert RR5 fälschlicherweise zu 960 ms bestimmt. Dadurch wird bei vier aufeinanderfolgenden Mittelwert-Bildungen 2. $\overline{RR}$ bis 5. $\overline{RR}$ der Mittelwert fälschlicherweise zu 600 ms bestimmt. Bei den Vergleichen 2. $\overline{PP}$/2. $\overline{RR}$ bis 5. $\overline{PP}$/5. $\overline{RR}$ (s. Fig. 5) wird dadurch für die Bewertung fälschlicherweise ermittelt, daß das ventrikuläre Herzintervall größer als das atriale Herzintervall ist. Dies wird als Kriterium dafür gewertet, daß eine atriale Tachykardie vorliegt.

Durch das erfindungsgemäße Korrekturverfahren werden neben den atrialen Herzintervall-Werten PP1, PP2 ... auch die ventrikuläre Herzintervalle RR1, RR2 ... einem Einzelvergleich unterzogen. Bei diesem Vergleich wird der falsche Herzintervall-Wert RR5 - wie bereits anhand von Fig. 2 und 3 bei den P-Wellen erläutert - erkannt und kann zu 480 ms korrigiert werden. Retrospektiv werden dann auch die gemittelten Herzintervall-Werte, wie sie in Fig. 5 noch falsch eingetragen sind, auch seitens der R-Wellen korrigiert, so daß auch die Mittelwerte 2. $\overline{RR}$ bis 5. $\overline{RR}$ zu 480 ms geändert werden. Die dann vorgenommenen Vergleiche 2. $\overline{PP}$/2. $\overline{RR}$ bis 5.$\overline{PP}$/5. $\overline{RR}$ führen zu dem Kriterium, daß atrial und ventrikulär gleiche Herzintervall-Werte vorliegen. Eine Fehlbewertung der Rhythmus durch unterschiedliche Intervalle wird also vermieden, somit kann das für atrial und ventrikulär gleiche Herzintervalle übliche sonstige Bewertungskriterium angewendet werden.

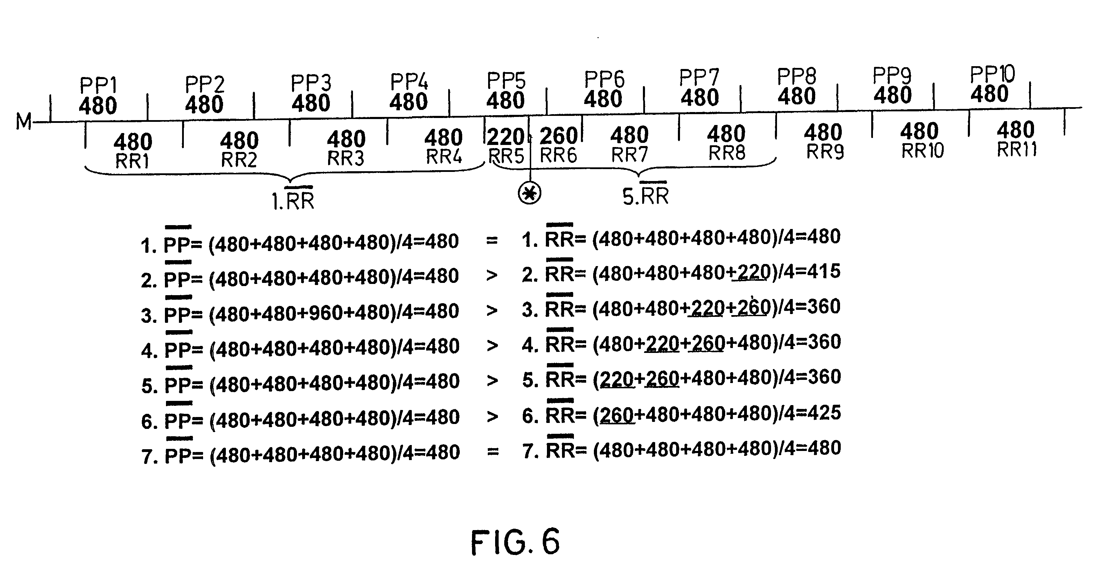

Die Fig. 6 zeigt ein vereinzeltes ventrikuläres "Oversensing", das sich durch die im Marker M mit einem Stern versehene, fälschlicherweise als R-Welle detektierte Zacke äußert. Aufgrund dieser Fehlmessung werden die ventrikulären Herzintervall-Werte RR5 und RR6 fälschlicherweise zu 220 bzw. 260 ms bestimmt. Bei der gleitenden Mittelwertbildung ergeben sich dadurch die falschen ventrikulären Herzintervall-Mittelwerte 2. $\overline{RR}$ bis 6. $\overline{RR}$. Die korrekt gemessenen atrialen Herzintervall-Werte PP1, PP2 ... führen zu korrekten Herzintervall-Mittelwerten 1. $\overline{PP}$, 2. $\overline{PP}$ usw.

In den Vergleichen 2. $\overline{PP}$/2. $\overline{RR}$ bis 6. $\overline{PP}$/6. $\overline{RR}$ wird aufgrund des ventrikulären "Oversensings" zwischen RR5 und RR6 ein falsches Verhältnis festgestellt, nämlich daß 2. $\overline{PP}$ bis 6. $\overline{PP}$ jeweils größer als 2. $\overline{RR}$ bis 6. $\overline{RR}$ sind. Dies führt zur falschen Annahme, daß eine ventrikuläre Tachykardie vorliegt.

Aufgrund des erfindungsgemäßen Verfahrens werden nun bei dem Einzelvergleich der Herzintervall-Werte Feststellungen dahingehend getroffen, daß die Werte RR5 und RR6 zwar signifikant kleiner als RR4 sind, der Wert RR7 jedoch wieder gleich dem ursprünglichen Wert RR4 ist. Dieser Umstand, nämlich daß genau zwei aufeinanderfolgende Herzintervall-Werte signifikant unter den vorherigen bzw. nachfolgenden Werten liegen, wird als Kriterium für eine Fehlmessung herangezogen und die beiden Herzintervall-Werte RR5 und RR6 werden nachfolgend zu einem einzigen korrekten Herzintervall-Wert mit einer Dauer von 480 ms korrigiert. Dies wirkt sich in der bereits erörterten Weise auf die gemittelten Herzintervall-Werte 2. $\overline{RR}$ bis 6. $\overline{RR}$ dahingehend aus, daß diese jeweils zu 480 ms korrigiert werden. Bei den anschließend neubewerteten Vergleichen 2. $\overline{PP}$/2. $\overline{RR}$ bis 6. $\overline{PP}$/6. $\overline{RR}$ wird damit festgestellt, daß die atrialen und ventrikulären Herzintervalle übereinstimmen, daß also ein sonst übliches Bewertungskriterium angewendet werden kann.

Ein vereinzeltes ventrikuläres "Oversensing" oder eine dazwischen liegende ventrikuläre Extrasystole (VES) werden durch den Korrekturalgorithmus gleichermaßen behandelt, obwohl eine VES ein reales Herzsignal darstellt. D. h., eine VES wird ebenfalls diskriminiert und bleibt unbewertet. Dieser Nebeneffekt ist gewollt, weil ventrikuläre Extrasystolen nicht behandlungspflichtig sind und damit das inadäquate Erfüllen eines Onset- oder Stabilitätskriteriums vermieden wird.

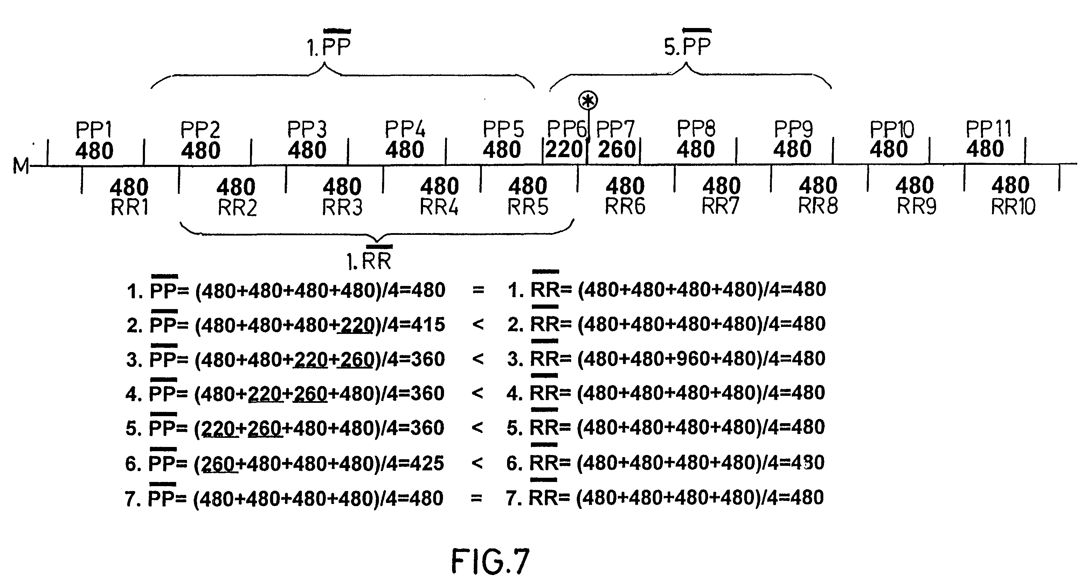

In Fig. 7 ist eine Darstellung analog Fig. 6 einer durch atriales "Oversensing" bedingten Fehlmessung dargestellt. Aufgrund der vorstehenden Ausführungen zum ventrikulären "Oversensing" ist diese Darstellung selbsterklärend und es wird zur Vermeidung von Wiederholungen auf die darauf unmittelbar übertragbaren Ausführungen zu Fig. 6 verwiesen.

## Patentansprüche

1. Verfahren zur Detektion kardialer Intervallsignale in kardiologischen Geräten, insbesondere zur Detektion von tachykarden Herzrhythmusstörungen, mit folgenden Verfahrensschritten:

   - zeitlich aufgelöstes Erfassen von kardialen Ereignissen, die für das Herzintervall repräsentativ sind,
   - Bestimmung des jeweiligen Herzintervalls zwischen zwei aufeinanderfolgenden kardialen Ereignissen,
   - statistische Auswertung einer bestimmten Anzahl aufeinanderfolgender Herzintervall-Werte,

   **dadurch gekennzeichnet,**
   **daß** einhergehend mit der statistischen Auswertung der bestimmten Anzahl von aufeinanderfolgenden Herzintervall-Werte ein Einzelvergleich dieser bestimmten Anzahl von Herzintervall-Werten untereinander derart erfolgt, daß einzelne signifikante, von vorher und danach auftretenden Herzintervall-Werten abweichende Herzintervall-Werte auf der Basis dieser statistischen Auswertung als Fehlmessungen erkannt und korrigiert werden.

2. Verfahren nach Anspruch 1, **dadurch gekennzeichnet, daß** bei überlagerter statistischer Auswertung und Korrektur der detektierten Herzintervall-Werte die statistisch ausgewerteten Herzintervall-Werte nachträglich korrigiert werden.

3. Verfahren nach Anspruch 1 oder 2, **dadurch gekennzeichnet, daß** als statistische Auswertung eine gleitende Mittelwertbildung über mindestens vier aufeinanderfolgende Herzintervall-Werte erfolgt.

4. Verfahren nach Anspruch 3, **dadurch gekennzeichnet, daß** der Einzelvergleich der Herzintervall-Werte untereinander mit einer Anzahl von Herzintervall-Werten erfolgt, die mit der für die Mittelwertbildung herangezogenen Anzahl von Herzintervall-Werten übereinstimmt.

5. Verfahren nach einem der Ansprüche 1 bis 4, **dadurch gekennzeichnet, daß** bei einem fehlerhaft nicht-festgestellten kardialen Ereignis genau ein Herzintervall-Wert signifikant von vorher und danach bestimmten Herzintervall-Werten abweicht und dieser abweichende Wert entsprechend auf diese Werte zu korrigieren ist.

6. Verfahren nach einem der Ansprüche 1 bis 4, **dadurch gekennzeichnet, daß** bei einem fehlerhaft festgestellten kardialen Ereignis genau zwei aufeinanderfolgende Herzintervall-Werte signifikant von dem vor und nach diesem Wertepaar bestimmten Herzintervall-Werte abweichen und entsprechend gemeinsam auf diese Werte zu korrigieren sind.

7. Verfahren nach einem der Ansprüche 1 bis 6, **dadurch gekennzeichnet, daß** als kardiale Ereignisse die atrial bedingte P-Welle und/oder die ventrikulär bedingte R-Welle des Elektrokardiogramms des überwachten Herzens erfaßt werden.

## Claims

1. A method for the detection of cardiac interval signals in cardiologic devices, in particular for the detection of cardiac arrhythmias, comprising the following method steps:

   - time-resolved detection of cardiac events which are representative of the cardiac interval;
   - determination of the respective cardiac interval between two successive cardiac events;
   - statistical evaluation of a certain number of successive cardiac interval values;

   **characterized**
   **in that** along with the statistical evaluation of the certain number of successive cardiac interval values, an individual comparison of this certain number of cardiac interval values with each other takes place such that individual, significant cardiac interval values, which deviate from cardiac interval values that occur there-before and thereafter, are recognized as faulty measurements and corrected on the basis of this statistic evaluation.

2. A method according to claim 1, **characterized in that** in the case of superimposed statistical evaluation and correction of the detected cardiac interval values, the statistically evaluated cardiac interval values are corrected subsequently.

3. A method according to claim 1 or 2, **characterized in that** sliding averaging of at least four successive cardiac interval values takes place as statistical evaluation.

4. A method according to claim 3, **characterized in that** the individual comparison of the cardiac interval values with each other is carried out for a number of cardiac interval values which is identical with the number of cardiac interval values used for sliding averaging.

5. A method according to one of claims 1 to 4, **characterized in that** in the case of faulty non-detection of a cardiac event, precisely one cardiac interval value deviates significantly from cardiac interval values determined there-before and there-after and **in that** this deviating value has to be corrected to correspond to these values.

6. A method according to one of claims 1 to 4, **characterized in that** in the case of faulty detection of a cardiac event, precisely two successive cardiac interval values deviate significantly from the cardiac interval values determined before and after this pair of values and have to be corrected jointly to correspond to these values.

7. A method according to one of the claims 1 to 6, **characterized in that** the atrially conditioned P wave and/or the ventricularly conditioned R wave of the electrocardiogram of the monitored heart are detected as cardiac events.

**Revendications**

1. Procédé de détection d'intervalles dans des signaux cardiaques dans des appareils cardiologiques, notamment de détection de perturbations tachycardes du rythme cardiaque, comprenant les étapes de procédé suivantes :

    - recensement séparé dans le temps d'événements cardiaques, qui sont représentatifs d'un intervalle cardiaque,
    - détermination de chaque intervalle cardiaque entre deux événements cardiaques successifs,

    - analyse statistique d'un nombre défini de valeurs successives d'intervalle cardiaque,

    **caractérisé en ce que**
    en association avec l'analyse statistique du nombre défini de valeurs successives d'intervalle cardiaque, une comparaison individuelle de ce nombre défini de valeurs d'intervalle cardiaque s'effectue de telle sorte que des valeurs d'intervalle cardiaque individuelles significatives, déviant des valeurs d'intervalle cardiaque apparaissant précédemment et ultérieurement, sur la base de cette analyse statistique, sont détectées comme des erreurs de mesure et sont corrigées.

2. Procédé selon la revendication 1, **caractérisé en ce que**, lorsque l'analyse statistique et la correction des valeurs détectées d'intervalle cardiaque se superposent, les valeurs d'intervalle cardiaque analysées de façon statistique sont corrigées ultérieurement.

3. Procédé selon la revendication 1 ou 2, **caractérisé en ce qu'**un établissement de la moyenne mobile s'effectue en tant qu'analyse statistique sur au moins quatre valeurs successives d'intervalle cardiaque.

4. Procédé selon la revendication 3, **caractérisé en ce que** la comparaison individuelle entre les valeurs d'intervalle cardiaque s'effectue avec un certain nombre de valeurs d'intervalle cardiaque, qui concorde avec le nombre de valeurs d'intervalle cardiaque dont on se sert pour établir la moyenne.

5. Procédé selon l'une quelconque des revendications 1 à 4, **caractérisé en ce que**, dans le cas d'un événement cardiaque incorrectement non déterminé, précisément une valeur d'intervalle cardiaque dévie des valeurs d'intervalle cardiaque définies précédemment et ultérieurement et cette valeur déviante doit être corrigée en fonction de ces valeurs.

6. Procédé selon l'une quelconque des revendications 1 à 4, **caractérisé en ce que**, dans le cas d'un événement cardiaque incorrectement déterminé, précisément deux valeurs successives d'intervalle cardiaque dévient de façon significative des valeurs d'intervalle cardiaque définies avant et après ce couple de valeurs et doivent être corrigées conjointement en fonction de ces valeurs.

7. Procédé selon l'une quelconque des revendications 1 à 6, **caractérisé en ce que** l'onde P provenant de l'atrium et/ou l'onde R provenant du ventricule, dans l'électrocardiogramme du coeur contrôlé, sont détectées comme des événements cardiaques.

FIG.1

M

P

P1  P2  P3...

R

R1  R2  R3...

EP 1 062 977 B1

8

7. $\overline{PP}$= 480

6. $\overline{PP}$= 600

5. $\overline{PP}$= 600

4. $\overline{PP}$= 600

3. $\overline{PP}$= 600

2. $\overline{PP}$= 480

1. $\overline{PP}$= 480

| PP1 480 | PP2 480 | PP3 480 | PP4 480 | PP5 960 | PP6 480 | PP7 480 | PP8 480 | PP9 480 |

M

| 480 RR1 | 480 RR2 | 480 RR3 | 480 RR4 | 480 RR5 | 480 RR6 | 480 RR7 | 480 RR8 | 480 RR9 | 480 RR10 |

1. $\overline{RR}$= 480

2. $\overline{RR}$= 480

3. $\overline{RR}$= 480

4. $\overline{RR}$= 480

5. $\overline{RR}$= 480

6. $\overline{RR}$= 480

7. $\overline{RR}$= 480

FIG.2

1. $\overline{PP}$= (480+480+480+480)/4=480    =    1. $\overline{RR}$= (480+480+480+480)/4=480

2. $\overline{PP}$= (480+480+480+480)/4=480    =    2. $\overline{RR}$= (480+480+480+480)/4=480

3. $\overline{PP}$= (480+480+960+<u>960</u>)/4=600    >    3. $\overline{RR}$= (480+480+480+480)/4=480

4. $\overline{PP}$= (480+480+<u>960</u>+480)/4=600    >    4. $\overline{RR}$= (480+480+480+480)/4=480

5. $\overline{PP}$= (480+<u>960</u>+480+480)/4=600    >    5. $\overline{RR}$= (480+480+480+480)/4=480

6. $\overline{PP}$= (<u>960</u>+480+480+480)/4=600    >    6. $\overline{RR}$= (480+480+480+480)/4=480

7. $\overline{PP}$= (480+480+480+480)/4=480    =    7. $\overline{RR}$= (480+480+480+480)/4=480

# FIG.3

FIG. 4

1. $\overline{PP}$= (480+480+480+480)/4=480    =    1. $\overline{RR}$= (480+480+480+480)/4=480

2. $\overline{PP}$= (480+480+480+480)/4=480    <    2. $\overline{RR}$= (480+480+480+960)/4=600

3. $\overline{PP}$= (480+480+960+480)/4=480    <    3. $\overline{RR}$= (480+480+960+480)/4=600

4. $\overline{PP}$= (480+480+480+480)/4=480    <    4. $\overline{RR}$= (480+960+480+480)/4=600

5. $\overline{PP}$= (480+480+480+480)/4=480    <    5. $\overline{RR}$= (960+480+480+480)/4=600

6. $\overline{PP}$= (480+480+480+480)/4=480    =    6. $\overline{RR}$= (480+480+480+480)/4=480

7. $\overline{PP}$= (480+480+480+480)/4=480    =    7. $\overline{RR}$= (480+480+480+480)/4=480

## FIG.5

PP1 480 | PP2 480 | PP3 480 | PP4 480 | PP5 480 | PP6 480 | PP7 480 | PP8 480 | PP9 480 | PP10 480

M

480 RR1 | 480 RR2 | 480 RR3 | 480 RR4 | 220 RR5 | 260 RR6 | 480 RR7 | 480 RR8 | 480 RR9 | 480 RR10 | 480 RR11

$\overline{1.RR}$        $\overline{5.RR}$

1. $\overline{PP}$= (480+480+480+480)/4=480    =    1. $\overline{RR}$= (480+480+480+480)/4=480

2. $\overline{PP}$= (480+480+480+480)/4=480    >    2. $\overline{RR}$= (480+480+480+220)/4=415

3. $\overline{PP}$= (480+480+960+480)/4=480    >    3. $\overline{RR}$= (480+480+220+260)/4=360

4. $\overline{PP}$= (480+480+480+480)/4=480    >    4. $\overline{RR}$= (480+220+260+480)/4=360

5. $\overline{PP}$= (480+480+480+480)/4=480    >    5. $\overline{RR}$= (220+260+480+480)/4=360

6. $\overline{PP}$= (480+480+480+480)/4=480    >    6. $\overline{RR}$= (260+480+480+480)/4=425

7. $\overline{PP}$= (480+480+480+480)/4=480    =    7. $\overline{RR}$= (480+480+480+480)/4=480

FIG. 6

EP 1 062 977 B1

FIG.7

$$1.\ \overline{PP}= (480+480+480+480)/4=480$$
$$2.\ \overline{PP}= (480+480+480+220)/4=415$$
$$3.\ \overline{PP}= (480+480+220+260)/4=360$$
$$4.\ \overline{PP}= (480+220+260+480)/4=360$$
$$5.\ \overline{PP}= (220+260+480+480)/4=360$$
$$6.\ \overline{PP}= (260+480+480+480)/4=425$$
$$7.\ \overline{PP}= (480+480+480+480)/4=480$$

$$1.\ \overline{RR}= (480+480+480+480)/4=480$$
$$2.\ \overline{RR}= (480+480+480+480)/4=480$$
$$3.\ \overline{RR}= (480+480+960+480)/4=480$$
$$4.\ \overline{RR}= (480+480+480+480)/4=480$$
$$5.\ \overline{RR}= (480+480+480+480)/4=480$$
$$6.\ \overline{RR}= (480+480+480+480)/4=430$$
$$7.\ \overline{RR}= (480+480+480+480)/4=480$$